# DEMANDE DE BREVET EUROPEEN

(11) **EP 1 661 545 A1**
(43) Date de publication de la demande: **31.05.2006**
(21) Numéro de dépôt: 05021193.7
(22) Date de dépôt: 28.09.2005
(51) Int. Cl.: A61K 8/06, A61K 8/34, A61K 8/36, A61Q 19/00

(54) **Crème à base d'acide gras sous forme d'émulsion H/E**

(30) Priorité: 15.10.2004 FR 0410946
(71) Demandeur: L'OREAL, 75008 Paris (FR)
(72) Inventeur: Terrisse, Isabelle, 94400 Vitry sur Seine (FR); Binutti, Béatrice, 92260 Fontenay aux Roses (FR)
(74) Mandataire: Rasson, Catherine

(57) **Abrégé**

L'invention se rapporte à une composition pour application topique, sous forme d'une émulsion huile-dans-eau comprenant une phase huileuse dispersée dans une phase aqueuse, caractérisée en ce qu'elle contient (1) au moins 10 % en poids par rapport au poids total de la composition, d'un ou plusieurs acides gras choisis parmi les acides gras saturés, linéaires ou ramifiés, comportant de 16 à 30 atomes de carbone, (2) au moins un alcool gras choisi parmi les alcools gras saturés, linéaires ou ramifiés, comportant de 22 à 40 atomes de carbone, et (3) au moins un agent basique en une quantité suffisante pour que la composition ait un pH allant de 6 à 9 et qu'au plus 90 % des acides gras soit sous forme libre, la composition contenant une quantité d'au plus 2 % en poids par rapport au poids total de la composition, d'acides gras saturés, linéaires ou ramifiés, comportant de 8 à 14 atomes de carbone.

La composition se présente sous forme d'un produit souple, notamment sous forme de crème.

La présente invention se rapporte aussi aux utilisations de ladite composition dans les domaines cosmétique et dermatologique, notamment pour le soin, la protection et/ou le maquillage de la peau et/ou des muqueuses.

L'invention concerne également un procédé de préparation de cette composition.

## Description

L'invention se rapporte à une composition sous forme d'une émulsion H/E se présentant sous forme de crème et contenant des savons comme tensioactifs, et à l'utilisation de la dite composition, en particulier pour le soin, la protection et/ou le maquillage de la peau du corps ou du visage, des cils et/ou des lèvres, et/ou pour le soin des cheveux.

L'invention concerne également un procédé de préparation de cette composition.

Il est connu de réaliser des compositions cosmétiques ou dermatologiques; constituant des crèmes de soin et se présentant sous forme d'émulsions H/E dans lesquelles l'émulsionnant est constitué d'acide gras au moins partiellement neutralisé. Ainsi, le document WO-02/19973 décrit des compositions de ce type. Ces crèmes à base de savons ne sont pas des produits moussants mais des crèmes de soin qui, outre leur aspect nacré esthétique, présentent l'avantage de donner un effet sensoriel cosmétique spécifique. En effet, elles ne laissent pratiquement pas de film huileux à la surface de la peau et se caractérisent par un toucher très sec, particulièrement apprécié dans les pays chauds. Toutefois, les compositions connues jusqu'à présent présentent l'inconvénient de ne pas pouvoir supporter des températures élevées, notamment supérieures à 40°C. Cela signifie que, si elles sont entreposées quelques jours à cette température, elles présentent une fluidification pouvant aboutir à une démixtion macroscopique finissant par une séparation en au moins deux phases. Les crèmes ainsi fluidifiées à une température nettement supérieure à la température ambiante se révèlent hétérogènes après retour à la température ambiante et donc inutilisables du fait de la dégradation de la texture. On entend ici par température ambiante, une température tempérée, c'est-à-dire d'environ 20 à 25°C.

Or, il est indispensable que ce type de produit soit stable sur une large plage de températures. En effet, au cours de sa vie, le produit peut être exposé à des températures allant jusqu'à 50°C ou plus selon les conditions de climat, de stockage et/ou de transport. Par exemple, il faut qu'une crème transportée dans une voiture qui risque de rester longtemps sous le soleil, c'est-à-dire à une température atteignant facilement 50°C et même 60°C, conserve sa stabilité. Il faut aussi que ces crèmes puissent être utilisées dans les pays chauds sans que leur transport et leur conservation ne posent de problème.

Pour améliorer leur stabilité, il est possible d'ajouter à ces émulsions, des émulsionnants hydrophiles, ces émulsionnants ayant généralement un HLB (Hydrophilic Lipophilic Balance) égal ou supérieur à 9. Toutefois, on essaie de plus en plus de limiter la quantité d'émulsionnant pour améliorer la tolérance des compositions sur la peau.

Une autre solution consiste à ajouter un gélifiant dans la phase aqueuse pour limiter la désorganisation du réseau cristallin quand la température augmente. Cette solution est préférable en théorie car les gélifiants sont généralement très peu sensibles à la température. Toutefois, l'ajout de tels gélifiants ne permet pas de résoudre ce problème comme le montrera un exemple comparatif présenté ci-après.

Il subsiste donc le besoin d'une émulsion H/E à base d'acide gras, stable jusqu'à au moins 60°C, qui ait une viscosité suffisante pour constituer une crème, dont l'aspect de crème soit maintenu à température ambiante même après passage à une température plus élevée, et qui ait une bonne stabilité, notamment une bonne stabilité de la viscosité (sans variation de viscosité après passage à une température plus élevée) et les propriétés requises pour un bon soin de la peau, et ce même en l'absence d'émulsionnant hydrophile, notamment d'émulsionnant hydrophile non ionique.

La demanderesse a découvert de manière surprenante qu'on pouvait atteindre le but de l'invention et obtenir une crème à base d'acide gras, ayant une bonne stabilité, même après passage à des températures atteignant 60°C, en utilisant au moins 10 % d'acide gras et en ajoutant un alcool gras ayant au moins 22 atomes de carbone tel que l'alcool béhénylique. En outre, la composition, même si elle peut contenir d'autres acides gras, doit contenir des acides gras donnant des savons insolubles, de préférence des acides en C16 à C22 tels que l'acide stéarique et l'acide palmitique. Par ailleurs, une partie des acides gras doit se trouver sous forme libre, c'est-à-dire ne pas être neutralisée, mais il ne doit pas y avoir plus de 90 % des acides gras sous forme libre.

Certes, il est connu d'ajouter des alcools gras dans les émulsions H/E contenant des acides gras, mais il a été trouvé ici que seul un alcool ayant au moins 22 atomes de carbone permettait d'atteindre le but de l'invention et que la présence d'un tel alcool était donc indispensable, même si d'autres alcools gras pouvaient être aussi présents, et que par ailleurs, la sélection d'acides gras était essentielle aussi pour atteindre le but de l'invention.

L'invention a donc pour objet une composition pour application topique, sous forme d'une émulsion huile-dans-eau (H/E) comprenant une phase huileuse dispersée dans une phase aqueuse, caractérisée en ce qu'elle contient (1) au moins 10 % en poids par rapport au poids total de la composition, d'un ou plusieurs acides gras choisis parmi les acides gras saturés, linéaires ou ramifiés, comportant de 16 à 30 atomes de carbone, (2) au moins un alcool gras choisi parmi les alcools gras saturés, linéaires ou ramifiés, comportant de 22 à 40 atomes de carbone, et (3) au moins un agent basique en une quantité suffisante pour que la composition ait un pH allant de 6 à 9 et qu'au plus 90 % des acides gras soit sous forme libre, la composition contenant une quantité d'au plus 2 % en poids par rapport au poids total de la composition, d'acides gras saturés, linéaires ou ramifiés, comportant de 8 à 14 atomes de carbone.

La composition selon l'invention étant destinée à une application topique, elle contient un milieu physiologiquement acceptable. On entend par "milieu physiologiquement acceptable", un milieu compatible avec la peau, les lèvres, les ongles, le cuir chevelu et/ou les cheveux.

La composition obtenue selon l'invention présente une bonne stabilité dans le temps, même à une température supérieure à la température ambiante (par exemple 50°C, voire même 60°C).

Par ailleurs, la composition selon l'invention présente l'avantage de donner un effet de matité sur la peau, et de pouvoir donc être utilisée comme composition matifiante.

La composition de l'invention se présente sous forme d'une crème plus ou moins fluide, c'est-à-dire un produit souple par opposition à un produit solide tel qu'un stick. Ainsi, cette composition a une viscosité à 25°C allant d'environ 10 à 150 poises (1 à 15 Pa.s) et de préférence d'environ 15 à 100 poises (1,5 à 10 Pa.s), cette viscosité étant mesurée avec un appareil Rhéomat 180 à 25°C avec les mobiles appropriés.

Selon un mode préféré de réalisation de l'invention, la composition selon l'invention est exempte d'émulsionnant hydrophile non ionique, c'est-à-dire ayant un HLB égal ou supérieur à 9, et/ou exempte de tensioactif amphotère hydrophile.

### Alcool gras

La composition selon l'invention contient au moins un alcool gras choisi parmi les alcools gras saturés, linéaires ou ramifiés, comportant de 22 à 40 atomes de carbone, de préférence 22 à 30 atomes de carbone. Il s'agit de préférence d'un alcool gras ayant une température de fusion supérieure à 60°C. L'alcool préféré est l'alcool béhénylique.

Le ou les alcools gras comportant de 22 à 40 atomes de carbone peuvent être par exemple présents en une quantité allant de 0,1 à 20 % en poids et de préférence de 0,5 à 10 % en poids et mieux de 0,5 à 5 % en poids par rapport au poids total de la composition.

En plus de l'alcool comportant de 22 à 40 atomes de carbone, la composition peut contenir un ou plusieurs alcools gras ayant de 12 à 21 atomes de carbone, tels que l'alcool cétylique, l'alcool stéarylique, et leurs mélanges (alcool cétéarylique). La quantité d'alcool(s) gras ayant de 12 à 21 atomes de carbone peut aller par exemple de 0,1 à 10 % en poids et mieux de 0,5 à 5 % en poids par rapport au poids total de la composition.

On peut éventuellement utiliser des matières premières dans lesquelles l'alcool comportant de 22 à 40 atomes de carbone, et notamment l'alcool béhénylique, est en mélange avec d'autres alcools gras.

### Acides gras

La composition selon l'invention contient un ou plusieurs acides gras choisis parmi les acides gras saturés, linéaires ou ramifiés, comportant de 16 à 30 atomes de carbone, et de préférence comportant de 16 à 22 atomes de carbone. Cet acide gras peut être en particulier choisi parmi l'acide palmitique, l'acide stéarique et leurs mélanges.

La quantité d'acide(s) gras comportant de 16 à 30 atomes de carbone (acides en C16-C30) est d'au moins 10 % et elle peut aller par exemple de 10 à 50 %, de préférence de 12 à 40 % en poids et mieux de 12 à 30 % en poids par rapport au poids total de la composition.

Une partie des acides gras est saponifiée (neutralisée) par l'agent basique, mais une partie doit rester sous forme d'acide gras libre. La quantité d'acide gras libre, c'est-à-dire non saponifiée, doit être au moins de 40 % et au plus de 90 % par rapport à la quantité totale d'acide gras. La quantité d'acide gras libre va de préférence de 40 à 90 % en poids et mieux de 50 à 90 % en poids par rapport à la quantité totale d'acide gras.

En plus des acides gras en C16-C30, la composition selon l'invention peut contenir des acides gras saturés, linéaires ou ramifiés, comportant de 8 à 14 atomes de carbone, tels que l'acide myristique et l'acide laurique. Toutefois, s'ils sont présents, ils ne doivent pas dépasser une certaine quantité bien inférieure à celle des acides en C16-C30. La quantité d'acides gras saturés, linéaires ou ramifiés, comportant de 8 à 14 atomes de carbone doit être inférieure ou égale à 2 % en poids par rapport au poids total de la composition ; cette quantité peut aller de 0 à 2 % en poids, et de préférence de 0,0001 à 2, et mieux de 0,0001 à 1,5 % en poids par rapport au poids total de la composition.

### Agent basique

Comme agents basiques, on peut utiliser par exemple les hydroxydes de métaux alcalins (hydroxyde de sodium et hydroxyde de potassium ou potasse), les hydroxydes de métaux alcalino-terreux (par exemple de magnésium), l'hydroxyde d'ammonium, ou encore les bases organiques comme la triéthanolamine, la N-méthylglucamine, la lysine et l'arginine.

L'agent basique est de préférence un hydroxyde de métal alcalin, et de préférence l'hydroxyde de potassium (KOH).

La quantité d'agent basique doit être suffisante pour que la composition ait un pH allant de 6 à 9 et de préférence de 7 à 8, et pour qu'une certaine quantité d'acide gras reste sous forme d'acide gras libre. Cette quantité d'agent basique peut aller par exemple de 0,1 à 10 % en poids, de préférence de 0,1 à 8 % en poids et mieux de 0,1 à 5 % en poids par rapport au poids total de la composition.

L'agent basique est de préférence présent dans la phase aqueuse.

### Phase aqueuse

La phase aqueuse de la composition de l'invention peut aller de 40 à 90 % en poids et de préférence de 50 à 85 % en poids par rapport au poids total de la composition. Elle contient au moins de l'eau qui peut constituer toute la phase aqueuse ou seulement une partie. Toutefois, la quantité d'eau est de préférence d'au moins 40 % en poids par rapport au poids total de la composition.

La phase aqueuse peut contenir, outre l'eau, un ou plusieurs solvants miscibles à l'eau ou au moins en partie miscibles à l'eau, comme les polyols ; les mono-alcools en C₂ à C₈, tels que l'éthanol et l'isopropanol ; et les cétones en C₃ à C₄ liquides à température ambiante. Par "température ambiante", il faut comprendre une température d'environ 25°C, à pression atmosphérique normale (760 mm de Hg).

Par "polyol", il faut comprendre toute molécule organique comportant au moins deux groupements hydroxyle libres. Comme polyols, on peut citer par exemple la glycérine, les glycols comme le butylène glycol, le propylène glycol, l'isoprène glycol, le dipropylène glycol, l'hexylène glycol, le pentylène glycol et les polyéthylène glycols comme le PEG-8, le sorbitol, les sucres comme le glucose.

Selon un mode préféré de réalisation de l'invention, la quantité de solvants miscibles à l'eau est de préférence inférieure à 10 %. Elle peut aller par exemple de 0,05 à 9,5 % en poids, de préférence de 0,1 à 9 % en poids et mieux de 0,5 à 8 % en poids par rapport au poids total de la composition.

### Phase huileuse

La phase huileuse peut être présente en une quantité allant de 10 à 60 % en poids par rapport au poids total de la composition. La phase huileuse est constituée des acides gras, des alcools gras et de tous les autres constituants lipophiles et notamment des huiles pouvant être présentes dans la composition de l'invention.

Selon un mode préféré de réalisation de l'invention, la phase huileuse contient au moins une huile. On entend par "huile" un corps gras liquide à la température ambiante (25°C).

Comme huiles utilisables dans la composition de l'invention, on peut citer par exemple :
- les huiles hydrocarbonées d'origine animale, telles que le perhydrosqualène (ou squalane) ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; les esters du pentaérythritol comme le tétraisostéarate de pentaérythrytyle ; les dérivés lipophiles d'acides aminés, tels que le lauroyl sarcosinate d'isopropyle (nom INCI : Isopropyl Lauroyl sarcosinate) commercialisé sous la dénomination Eldew SL 205 par la société Ajinomoto ;
- les hydrocarbures linéaires ou ramifiés, d'origine minérale ou synthétique, tels que les huiles minérales (mélange d'huiles hydrocarbonées dérivées du pétrole ; nom INCI : Mineral oil), les huiles de paraffine, volatiles ou non, et leurs dérivés, la vaseline, les polydécènes, l'isohexadecane, l'isododecane, l'isoparaffine hydrogéné tel que l'huile de Parléam® commercialisée par la société NOF Corporation (nom INCI ; Hydrogenated Polyisobutene) ;
- leurs mélanges.

Selon un mode préféré de réalisation de l'invention, la phase huileuse de la composition contient de préférence au moins une huile choisie parmi les hydrocarbures ramifiés, d'origine minérale ou synthétique, tels que l'isohexadecane, l'isododecane, l'isoparaffine hydrogéné tel que l'huile de

Parléam®, et leurs mélanges.

La quantité d'huiles peut aller par exemple de 0,1 à 10 %, mieux de 0,5 à 5 % par rapport au poids total de la composition.

Les quantités d'acides gras et d'alcools gras sont telles qu »indiquées plus haut.

### Adjuvants

De façon connue, la composition de l'invention peut contenir également des adjuvants habituels dans les domaines cosmétique et/ou dermatologique, tels que les actifs, les conservateurs, les antioxydants et anti-radicaux libres, les agents complexants, les parfums, les charges, les bactéricides, les absorbeurs d'odeur, les matières colorantes (pigments et colorants) et encore les vésicules lipidiques. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse et/ou dans les vésicules lipidiques.

Comme charges qui peuvent être utilisées dans la composition de l'invention, on peut citer par exemple, le talc ; les oxydes de zinc ; les oxydes de titane tels que le dioxyde de titane ; les micas d'origine naturelle ou synthétique ; le carbonate de calcium ; le carbonate et l'hydrocarbonate de magnésium ; la silice, en particulier la silice sphérique, la poudre de silice commercialisée sous la dénomination Cab-O-Sil TS 530 par la société Cabot, et les microbilles de silice telles que celles commercialisées sous la dénomination SB150 par la société Myoshi ; le kaolin ; les billes de verre et de céramique commercialisées par la société 3M sous la dénomination commerciale MACROLITE ; les savons métalliques dérivés d'acide organique carboxylique ayant de 8 à 22 atomes de carbone, de préférence de 12 à 18 atomes de carbone, par exemple le stéarate de zinc, de magnésium ou de lithium, le laurate de zinc, le myristate de magnésium ; les particules de polyamide et notamment celles vendues sous les dénominations ORGASOL par la société Atochem ; les poudres de polymères synthétiques non expansées, telles que les poudres de polyéthylène, de polystyrène, de polyesters, de polyamides (par exemple le nylon ou la poly-β-alanine), de copolymères d'acrylates (par exemple les microsphères microporeuses vendues par la société Dow Corning sous la dénomination commerciale POLYTRAP), d'acides polyméthacryliques, de Téflon® (polytétrafluuoroéthylène) comme les produits commercialisés sous les dénominations FLUON par la société Uniqema ; les poudres expansées telles que les microsphères creuses en matériau thermoplastique préparées par des procédés connus, comme ceux décrits dans les documents US-A-3 615 972 et EP-A-0 56219, et notamment, les microsphères commercialisées sous les dénominations EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone ; les fibres ; et leurs mélanges.

Quand elles sont présentes, ces charges peuvent être en une quantité allant par exemple de 0,01 à 10 % en poids, de préférence de 0,1 à 8 % en poids et mieux de 0,5 à 5 % en poids par rapport au poids total de la composition.

Comme actifs utilisables dans la composition de l'invention, on peut citer par exemple, les enzymes (par exemple lactoperoxydase, lipase, protéase, phospholipase, cellulases) ; les flavonoïdes ; les agents hydratants tels que les hydrolysats de protéines ; le hyaluronate de sodium ; les polyols comme la glycérine, les glycols comme les polyéthylène glycols, et les dérivés de sucre ; les anti-inflammatoires ; les oligomères procyannidoliques ; les vitamines comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; l'urée ; la caféine ; les dépigmentants tels que l'acide kojique, l'hydroquinone et l'acide caféique ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A ; les filtres solaires ; l'hydrocortisone ; la mélatonine ; les extraits d'algues, de champignons, de végétaux, de levures, de bactéries ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents matifiants ; les agents tenseurs ; les céramides ; les huiles essentielles ; et leurs mélanges ; et tout actif approprié pour le but final de la composition.

Les filtres solaires peuvent être choisis parmi les filtres UV organiques, tels que les composés suivants :
- Les dérivés salicyliques et notamment l'éthylhexyl salicylate (ou salicylate d'éthyl hexyle) vendu sous le nom commercial NEO HELIOPAN OS par HAARMANN et REIMER ;
- Les dérivés du dibenzoylméthane et notamment le Butyl méthoxydibenzoylmethane vendu notamment sous le nom commercial PARSOL 1789 par HOFFMANN LA ROCHE ;
- Les dérivés cinnamiques et notamment l'éthylhexyl méthoxycinnamate vendu notamment sous le nom commercial PARSOL MCX par HOFFMANN LA ROCHE;
- Les dérivés de β,β'-diphénylacrylate et notamment l'octocrylène (α-cyano-β,β-diphénylacrylate de 2-éthylhexyle) vendu notamment sous le nom commercial UVINUL N539 par BASF ;
- L'acide phenylbenzimidazole sulfonique ;
- Les dérivés du benzylidène camphre, et notamment l'acide téréphthalylidène dicamphosulfonique (Terephthalylidene Dicamphor Sulfonic) fabriqué sous le nom MEXORYL SX par CHIMEX, et le 4-méthylbenzylidène camphre vendu sous le nom commercial EUSOLEX 6300 par MERCK ;
- Les dérivés de la benzophénone, et notamment la Benzophenone-3 ou Oxybenzone, vendue sous le nom commercial UVINUL M40 par BASF ; la Benzophenone-4 vendue sous le nom commercial UVINUL MS40 par BASF ; la Benzophenone-5 ;
- Les dérivés du phényl benzimidazole, et notamment le Benzimidazilate vendu sous le nom commercial commercial NEO HELIOPAN AP par HAARMANN et REIMER ;
- Les dérivés de la triazine, et notamment l'anisotriazine vendu sous le nom commercial TINOSORB S par CIBA GEIGY ; l'éthylhexyl triazone vendu notamment sous le nom commercial UVINUL T150 par BASF ; et le diéthylhexyl Butamido Triazone vendu sous le nom commercial UVASORB HEB par SIGMA 3V ;
- Le méthylène bis-benzotriazolyl tétraméthylbutylphenol ;
- Les dérivés du phényl benzotriazole, et notamment le Drometrizole Trisiloxane vendu sous le nom commercial SILATRIZOLE par RHODIA CHIMIE ;
- et leurs mélanges.

Les filtres solaires peuvent être choisis aussi parmi les filtres physiques. Comme filtres physiques pouvant être ajoutés dans la composition de l'invention, on peut citer par exemple les pigments et nano-pigments d'oxydes métalliques, enrobés ou non enrobés, notamment les oxydes de titane, de fer, de zirconium, de zinc ou de cérium, et leurs mélanges, ces oxydes pouvant être sous forme de micro- ou nanoparticules (nanopigments), éventuellement enrobées.

La quantité d'actifs dépend du but recherché. Le ou les actifs peuvent être par exemple présents en une concentration allant de 0,001 à 10 % et de préférence de 0,01 à 5 % du poids total de la composition.

Par ailleurs, la composition selon l'invention peut éventuellement contenir un ou plusieurs polymères gélifiants tels que les polymères carboxyvinyliques, les polyacrylamides ou les polysaccharides. Mais l'addition d'un tel polymère n'est pas nécessaire pour avoir une bonne stabilité. La quantité de ces polymères peut aller par exemple de 0,005 à 2 % en poids et mieux de 0,01 à 1 % en poids par rapport au poids total de la composition. Toutefois, la composition peut être exempte de polymère gélifiant.

Bien entendu, l'homme de l'art veillera à choisir le ou les éventuels composés à ajouter à la composition selon l'invention et leurs quantités, de manière telle que les propriétés avantageuses attachées intrinsèquement à la composition conforme à l'invention ne soient pas ou substantiellement pas, altérées par l'addition envisagée.

La composition selon l'invention peut se présenter notamment sous forme d'une crème plus ou moins fluide, et elle peut constituer notamment une composition cosmétique ou dermatologique. Elle trouve alors son application dans un grand nombre de traitements notamment cosmétiques de la peau, y compris du cuir chevelu, des cheveux, des ongles, et/ou des muqueuses, en particulier pour le soin, la protection et/ou le maquillage de la peau du corps ou du visage, des cils et/ou des lèvres, et/ou pour le soin des cheveux.

Aussi, la présente invention a pour objet l'utilisation cosmétique d'une composition cosmétique telle que définie ci-dessus, pour le soin, la protection et/ou le maquillage de la peau et/ou des lèvres et/ou pour le soin des cheveux.

La présente invention a encore pour objet un procédé de traitement cosmétique de la peau, y compris du cuir chevelu, des cheveux, et/ou des lèvres, caractérisé par le fait que l'on applique sur la peau, les cheveux et/ou les lèvres, une composition cosmétique telle que définie ci-dessus.

Par ailleurs, ainsi qu'indiqué plus haut, la composition selon l'invention présente l'avantage de pouvoir donc être utilisée comme composition matifiante. Les compositions matifiantes sont utilisées pour éviter la brillance de la peau qui est souvent liée à une sécrétion importante de sébum, problème affectant plus particulièrement les adolescents mais pouvant aussi se manifester à l'âge adulte sous l'effet notamment d'une hyperproduction d'androgènes. Cette brillance peut aussi être liée à la sueur résultant d'une activité physique ou des conditions climatiques. Or, une peau brillante est considérée comme inesthétique, d'autant plus qu'elle entraîne souvent une moins bonne tenue du maquillage qui a tendance à se dégrader visuellement au cours de la journée.

Aussi, la présente invention a pour objet l'utilisation cosmétique d'une composition cosmétique telle que définie ci-dessus, pour matifier la peau, c'est-à-dire donner un aspect mat à la peau, notamment donner un aspect mat à la peau qui brille.

La composition selon l'invention peut être préparée de manière avantageuse en utilisant un procédé particulier, procédé consistant à chauffer les acides gras et alcools gras (par exemple à une température d'environ 70°C à 90°C), à y mélanger le reste de la phase huileuse, à verser la phase huileuse dans la phase aqueuse ne contenant pas l'agent basique, et à mélanger sous forte agitation, c'est-à-dire à des vitesses d'agitation allant d'environ 1 000 à 10 000 tr/min, par exemple à l'aide d'un émulseur, pendant un temps suffisant pour que l'émulsion se forme, par exemple 5 à 10 minutes, pour obtenir une émulsion fine, puis à ajouter l'agent basique en agitant lentement pour que la saponification se produise, et à refroidir avant d'ajouter éventuellement les charges ou les actifs thermosensibles.

Un autre objet de l'invention est donc un procédé de préparation d'une composition selon l'invention, caractérisé en ce qu'on chauffe les acides gras et les alcools gras, on y mélange le reste de la phase huileuse, on verse la phase huileuse dans la phase aqueuse ne contenant pas l'agent basique et on mélange sous forte agitation, on ajoute ensuite l'agent basique en agitant lentement pour que la saponification se produise, et on refroidit avant d'ajouter éventuellement les charges ou les actifs thermosensibles.

Les exemples ci-après de compositions selon l'invention sont donnés à titre d'illustration et sans caractère limitatif. Les quantités y sont données en % en poids, sauf mention contraire.

Exemple 1 selon l'invention

### Phase A1

- Acide stéarique 9 %
- Acide palmitique 7,5 %
- Acide myristique 0,5 %
- Alcool béhénylique 1 %
- Alcool cétylique 1 %

### Phase A2

- Isohexadecane 2 %

### Phase B

- Glycérine 2 %
- conservateur (méthylparaben + phenoxyéthanol) 1 %
- Eau qsp 100 %

### Phase C

| | |
|---|---|
| Hydroxyde de potassium (KOH) | 1,2 % |

### Mode opératoire :

On chauffe la phase A1 à 80°C dans une cuve équipée d'un émulseur et on ajoute la phase A2 également portée à 80°C, puis on verse le mélange dans la phase B préalablement mélangée et portée à 80°C en agitant sous forte vitesse pendant 2 minutes. Ensuite, on introduit KOH en mélangeant à l'aide de pales pendant 10 minutes, et on refroidit.

L'émulsion H/E obtenue se présente sous forme d'une crème épaisse blanche d'aspect nacré. Elle reste stable après un mois à des températures à 4°C, à 25°C et à 45°C. Elle peut être utilisée comme crème de soin de la peau à laquelle elle apporte un toucher sec très spécifique.

Exemple 2 selon l'invention

### Phase A1

- Acide stéarique 9 %
- Acide palmitique 7,5 %
- Acide myristique 0,5 %
- Alcool béhénylique 1 %
- Alcool cétylique 1 %

### Phase A2

- Isohexadecane 2 %

### Phase B

- Glycérine 2 %
- conservateur (méthylparaben + phenoxyéthanol) 1 %
- Eau qsp 100 %

### Phase C

| | |
|---|---|
| Hydroxyde de potassium (KOH) | 1,2 % |

### Phase D

| | |
|---|---|
| Oxyde de titane | 2 % |

### Mode opératoire :

On chauffe la phase A1 à 80°C dans une cuve équipée d'un émulseur et on ajoute la phase A2 également portée à 80°C, puis on verse le mélange dans la phase B préalablement mélangée et portée à 80°C en agitant sous forte vitesse pendant 2 minutes. Ensuite, on introduit KOH en mélangeant à l'aide de pales pendant 10 minutes, et on refroidit. Ensuite, on ajoute la phase D et on homogénéise pour obtenir une crème lisse et homogène.

L'émulsion H/E obtenue se présente sous forme d'une crème épaisse blanche. Elle reste stable après un mois à des températures à 4°C, à 25°C et à 45°. Elle peut être utilisée comme crème de soin de la peau à laquelle elle apporte un toucher sec très spécifique.

Exemple 3 selon l'invention et exemples comparatifs 1 et 2 : Les exemples comparatifs se différencient de l'exemple 3 par le fait qu'ils ne contiennent pas d'alcool béhénylique, ce dernier ayant été remplacé et par de l'alcool cétylique ou cétylique et cétéarylique. En plus, l'exemple comparatif 3 contient un gélifiant.

| Composition | Exemple 3 selon l'invention | Exemple comparatif 1 | Exemple comparatif 2 | Exemple comparatif 3 |
|---|---|---|---|---|
| Phase A1 | | | | |
| Acide stéarique | 17 | 17 | 17 | 17 |
| Alcool béhénylique | 1 | 0 | 0 | 0 |
| Alcool cétéarylique | 0 | 0 | 0 | 1 |
| Alcool cétylique | 1 | 0,44 | 2 | 1 |

| Phase A2 | | | | |
|---|---|---|---|---|
| Isohexadecane | 2 | 0 | 2 | 2 |

| Phase B | | | | |
|---|---|---|---|---|
| Glycérine | 2 | 2 | 2 | 2 |
| conservateur | 1 | 1 | 1 | 1 |
| Hydroxypropylcellulose | 0 | 0 | 0 | 0,5 |
| Eau | qsp 100 | qsp 100 | qsp 100 | qsp 100 |

| *Phase* C | | | | |
|---|---|---|---|---|
| KOH | 1,2 | 1,2 | 1,2 | 1,2 |

| Stabilité | | | | |
|---|---|---|---|---|
| Viscosité à Température ambiante (25°C) | 52 Poises | 19 Poises | 52 poises | 59 poises |
| Viscosité après 1 mois à 45°C puis retour à température ambiante | 52 Poises | Plus de 40 Poises | 59 Poises | 71 Poises |

Les viscosités ont été mesurées au Rhéomat RM180 après 30 secondes de cisaillement.

Ce tableau met en évidence une augmentation de la valeur de la viscosité (après retour à la température ambiante après conservation pendant un mois à 45°C) par rapport à la viscosité initiale à la température ambiante pour les compositions ne contenant pas d'alcool béhénylique, alors que la composition selon l'invention (exemple 3) présente une viscosité qui reste stable au retour à la température ambiante après conservation pendant un mois à 45°C.

En outre, l'exemple comparatif 3 montre que l'ajout d'un agent gélifiant tel que l'hydroxypropylcellulose épaissit la composition mais n'améliore pas la stabilité de la composition en cas de variation de température.

## Revendications

1. Composition pour application topique, sous forme d'une émulsion huile-dans-eau, comprenant une phase huileuse dispersée dans une phase aqueuse, **caractérisée en ce qu'**elle contient (1) au moins 10 % en poids par rapport au poids total de la composition, d'un ou plusieurs acides gras choisis parmi les acides gras saturés, linéaires ou ramifiés, comportant de 16 à 30 atomes de carbone, (2) au moins un alcool gras choisi parmi les alcools gras saturés, linéaires ou ramifiés, comportant de 22 à 40 atomes de carbone, et (3) au moins un agent basique en une quantité suffisante pour que la composition ait un pH allant de 6 à 9 et qu'au plus 90 % des acides gras soit sous forme libre, la composition contenant une quantité d'au plus 2 % en poids par rapport au poids total de la composition, d'acides gras saturés, linéaires ou ramifiés, comportant de 8 à 14 atomes de carbone.

2. Composition selon la revendication 1, **caractérisée en ce que** l'alcool gras comportant de 22 à 40 atomes de carbone est l'alcool béhénylique.

3. Composition selon la revendication 1 ou 2, **caractérisée en ce que** l'alcool gras comportant de 22 à 40 atomes de carbone est présent en une quantité allant de 0,1 à 20 % en poids par rapport au poids total de la composition.

4. Composition selon l'une quelconque des revendications précédentes, **caractérisée en ce qu'**elle comprend en outre un ou plusieurs alcools gras ayant de 12 à 21 atomes de carbone.

5. Composition selon l'une quelconque des revendications précédentes, **caractérisée en ce que** l'acide gras comportant de 16 à 22 atomes de carbone est choisi parmi l'acide palmitique, l'acide stéarique et leurs mélanges.

6. Composition selon l'une quelconque des revendications précédentes, **caractérisée en ce que** la quantité d'acide(s) gras comportant de 16 à 22 atomes de carbone va de 10 à 50 % et de préférence de 12 à 40 % en poids par rapport au poids total de la composition.

7. Composition selon l'une quelconque des revendications précédentes, **caractérisée en ce que** l'agent basique est choisi parmi les hydroxydes de métaux alcalins, les hydroxydes de métaux alcalino-terreux, l'hydroxyde d'ammonium et les bases organiques.

8. Composition selon l'une quelconque des revendications précédentes, **caractérisée en ce que** la quantité d'agent basique va de 0,1 à 10 % en poids et de préférence de 0,1 à 8 % en poids par rapport au poids total de la composition.

9. Composition selon l'une quelconque des revendications précédentes, **caractérisée en ce que** la quantité d'acide gras libre va de 40 à 90 % en poids et de préférence de 50 à 90 % en poids par rapport à la quantité totale d'acide gras.

10. Composition selon l'une quelconque des revendications précédentes, **caractérisée en ce que** la phase aqueuse va de 40 à 90 % en poids par rapport au poids total de la composition.

11. Composition selon l'une quelconque des revendications précédentes, **caractérisée en ce que** la phase huileuse contient au moins une huile choisie parmi les hydrocarbures ramifiés, d'origine minérale ou synthétique.

12. Composition selon l'une quelconque des revendications précédentes, **caractérisée en ce qu'**elle constitue une composition cosmétique ou dermatologique.

13. Utilisation cosmétique d'une composition cosmétique selon l'une quelconque des revendications 1 à 11, pour le soin, la protection et/ou le maquillage de la peau et/ou des lèvres et/ou pour le soin des cheveux.

14. Utilisation cosmétique d'une composition cosmétique selon l'une quelconque des revendications 1 à 11, pour matifier la peau.

15. Procédé de traitement cosmétique de la peau, y compris du cuir chevelu, des cheveux, et/ou des lèvres, **caractérisé en ce que** l'on applique sur la peau, les cheveux et/ou les lèvres, une composition cosmétique selon l'une quelconque des revendications 1 à 11.

16. Procédé de préparation d'une composition selon l'une quelconque des revendications 1 à 11, **caractérisé en ce qu'**on chauffe les acides gras et les alcools gras, on y mélange le reste de la phase huileuse, on verse la phase huileuse dans la phase aqueuse ne contenant pas l'agent basique et on mélange sous forte agitation, on ajoute ensuite l'agent basique en agitant lentement pour que la saponification se produise, et on refroidit avant d'ajouter éventuellement les charges ou les actifs thermosensibles.
